(19) Europäisches Patentamt
European Patent Office
Office européen des brevets

(11) Veröffentlichungsnummer: **0 036 131**
**B1**

(12) # EUROPÄISCHE PATENTSCHRIFT

(45) Veröffentlichungstag der Patentschrift: **27.06.84**

(51) Int. Cl.³: **C 07 D 307/32**

(21) Anmeldenummer: **81101540.3**

(22) Anmeldetag: **04.03.81**

(54) **Verfahren zur Herstellung von Butyrolactonen.**

(30) Priorität: **13.03.80 DE 3009604**

(43) Veröffentlichungstag der Anmeldung: **23.09.81 Patentblatt 81/38**

(45) Bekanntmachung des Hinweises auf die Patenterteilung: **27.06.84 Patentblatt 84/26**

(84) Benannte Vertragsstaaten: **CH DE FR GB LI**

(56) Entgegenhaltungen:
**EP - A - 0 018 162**
**US - A - 3 868 370**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Van-Leyden-Strasse 17, D-6700 Ludwigshafen (DE)**

**EP 0 036 131 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Diese Erfindung betrifft ein Verfahren zur Herstellung von Butyrolactonen durch Umsetzung von $\gamma$-Formylcarbonsäureestern mit Sauerstoff bei höherer Temperatur.

Butylrolactone sind wertvolle und vielseitige Zwischenprodukte, die z. B. für die Herstellung von Herbiziden und Pharmazeutika Verwendung finden (siehe z. B. DE-OS 2 715 284 und DE-OS 2 715 133).

Man kann Butyrolactone z. B. durch intramolekulare Cyclisierung von $\gamma$-Hydroxy- oder $\gamma$-Halogencarbonsäuren oder deren Derivaten herstellen. Diese in Houben-Weyl, »Methoden der Organischen Chemie«, 1963, Band 6/2, Seiten 571 bis 580 beschriebene Synthese hat wegen Schwierigkeiten bei der Herstellung der erforderlichen Ausgangsstoffe nur in wenigen speziellen Fällen technische Bedeutung erlangt.

Darüber hinaus lassen sich Butyrolactone z. B. herstellen durch säurekatalysierte Cyclisierung von 4-Acetoxibuttersäure bei einer Temperatur von bis zu 250° C (US-PS 3 868 370); Umsetzung von Malonsäureestern mit Epoxiden in Gegenwart von Alkalialkoholaten (US-PS 3 299 100); Reduktion von 1,2-Dicarbonsäureanhydriden in Gegenwart von speziellen Rutheniumkatalysatoren (US-PS 3 957 827); Umsetzung von Bromessigsäure bzw. ihren Säurederivaten mit $\alpha$-Olefinen bei hoher Temperatur in Gegenwart von Radialbildnern (US-PS 2 986 568); Behandlung von $\gamma$-Halogenalkoholen mit Blausäure oder Cyaniden in Gegenwart von Phosphinen (US-PS 3 963 757), Umsetzung von in $\alpha$-Stellung monoverzweigten aliphatischen Aldehyden mit Kohlenmonoxid in Gegenwart von sauren Katalysatoren (DE-PS 1 192 178); oder durch Umsetzung von $\beta$-Olefinen mit Formaldehyd oder Acetaldehyd und Kohlenmonoxid in Gegenwart von Rhodiumchlorid unter hohem Druck und hoher Temperatur (US-PS 3 952 020).

Diese bekannten Verfahren haben den Nachteil, daß sie von teuren und schwer zugänglichen Verbindungen ausgehen (US-PS 3 299 100, US-PS 3 957 827, US-PS 2 986 568) und wegen der Verwendung von gefährlichen Ausgangsprodukten wie Blausäure, Cyanide und Kohlenmonoxid, eine aufwendige Technik erfordern (US-PS 3 963 757, US-PS 3 952 020, DE-PS 1 192 178). Drastische Bedingungen, wie hohe Drücke und hohe Temperaturen komplizieren in vielen Fällen die technische Durchführung und verbieten den Einsatz von Ausgangsstoffen mit wertvollen Substituenten, wie Cyanid- und Carboxylgruppen. Die Verfahren liefern außerdem zum Teil Gemische von isomeren Produkten, deren Trennung sehr schwierig ist.

Es wurde nun überraschend gefunden, daß man Butyrolactone der allgemeinen Formel

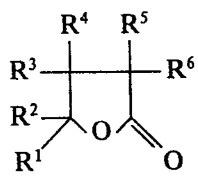

(I)

in der $R^1$ eine gegebenenfalls durch Cyan-, Alkoxycarbonyl-, Alkylcarbonyloxy- oder Nitrogruppen oder Chloratome substituierte Alkyl- oder Phenylgruppe und die Reste $R^2$ bis $R^6$ Wasserstoffatome oder gegebenenfalls durch Cyan-, Alkoxycarbonyl-, Alkylcarbonyloxy- oder Nitrogruppen oder Chloratome substituierte Alkyl- oder Phenylgruppen bedeuten, wobei die Alkylgruppen $R^1$ bis $R^6$ 1—8 C-Atome aufweisen, besonders vorteilhaft dadurch herstellen kann, daß man $\gamma$-Formylcarbonsäureester der allgemeinen Formel

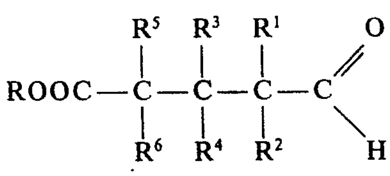

(II)

in der R eine niedere Alkylgruppe mit bis zu 4 C-Atomen bedeutet, bei 60 bis 150° C mit Sauerstoff behandelt.

Nach dem Verfahren der Erfindung erhält man die Butyrolactone glatt und in guter Ausbeute. Dieses Ergebnis ist sehr überraschend, da allgemein bekannt ist, daß die Oxidation von Aldehyden mit Sauerstoff hauptsächlich zu den entsprechenden Carbonsäuren bzw. Anhydriden führt (Houben-Weyl, Methoden der Organischen Chemie, 1952, Band 8, Seite 24).

In den $\gamma$-Formylcarbonsäureestern der Formel II können die Alkyl- und die Phenylgruppen $R^1$ bis $R^6$ eine oder mehrere Cyan-, Alkoxycarbonyl-, Alkylcarbonyloxy- oder Nitrogruppen oder Chloratome enthalten. Die Alkylgruppen $R^1$ bis $R^6$ haben 1 bis 8, vorzugsweise 1 bis 6 C-Atome. Die niedere Alkylgruppe R ist eine Alkylgruppe mit bis 4 C-Atomen. Besonders bevorzugt sind solche $\gamma$-Formylcarbonsäureester der Formel II, in der R Methyl, Ethyl, Propyl oder Butyl, die Reste $R^1$ und $R^2$ Alkylgruppen mit 1 bis 6 C-Atomen, die z. B. durch Cyano-, Methoxycarbonyl-, Acetoxy-, Nitro- oder Phenylgruppen oder durch Chloratome substituiert sein können, und die Reste $R^3$ bis $R^6$ Wasserstoffatome bedeuten.

Als Ausgangsstoffe seien beispielsweise genannt:

4-Formyl-4-methyl-valeriansäure-methyl- oder -ethylester,
4-Formyl-4-ethylvaleriansäure-methyl- oder -ethylester,
4-Formyl-4-propylvaleriansäuremethyl- oder -ethylester,
4-Formyl-4-butylvaleriansäuremethyl- oder -ethylester,
4-Formyl-4-ethyl-hexansäuremethylester, 4-Formyl-4-ethyl-heptansäuremethylester,
4-Formyl-4-propyl-hexansäuremethylester,
4-Formyl-3,4-dimethyl-valeriansäuremethyl- oder -ethylester,
4-Formyl-2,4-dimethylvaleriansäuremethylester,
4-Formyl-4-($\beta$-cyanoethyl)-hexansäuremethylester, und
4-Formyl-4-($\beta$-methoxycarbonylethyl)-hexansäuremethylester.

Im Gegensatz zu den bekannten Verfahren läßt sich das erfindungsgemäße Verfahren technisch äußerst einfach durchführen. Die Ausgangsstoffe sind leicht zugänglich. Beispielsweise können die benötigten $\gamma$-Formylcarbonsäureester nach bekannten Verfahren aus den entsprechenden Aldehyden oder Enaminen und $\alpha,\beta$-ungesättigten Estern hergestellt werden (Organic Reactions, Vol. 10, Seite 179).

Das neue Verfahren wird im allgemeinen so durchgeführt, daß man in den $\gamma$-Formylcarbonsäureester, vorteilhaft unter Rühren bei 60 bis 150° C Sauerstoff einleitet. Man kann dabei sowohl technischen Sauerstoff als auch Luft oder ein anderes Sauerstoff-Inertgas-Gemisch verwenden. Die Gaseinleitgeschwindigkeit beträgt zweckmäßigerweise 5 bis 50 l/Stunde und die Sauerstoffmenge 2 bis 10 Mol/Mol Formylcarbonsäureester. Die Umsetzung ist nach 2 bis 10 Stunden beendet. Man arbeitet zweckmäßig ohne Lösungsmittel, es kann jedoch auch in Gegenwart eines inerten Lösungsmittels, wie beispielsweise Benzol, Chlorbenzol oder Nitromethan gearbeitet werden.

Im allgemeinen wird die Umsetzung ohne Katalysator durchgeführt. Um höhere Reaktionsgeschwindigkeiten und bessere Selektivitäten zu erreichen, kann es mitunter zweckmäßig sein, in Gegenwart eines Katalysators zu arbeiten. Als Katalysatoren sind beispielsweise Schwermetallsalze wie $NiCl_2$, $Ni(OAc)_2$, $VCl_3$, $CrCl_3$, $CoCl_2$ oder $CeCl_3$ geeignet. Die Menge des Katalystators ist nicht kritisch, sie beträgt im allgemeinen 0,01 bis 0,1 Molprozent, bezogen auf das Ausgangsprodukt. Ob ein Katalysator überhaupt notwendig ist und welcher Katalysator zweckmäßig ist, kann jeweils leicht durch einen Vorversuch ermittelt werden.

Die Umsetzungen können sowohl drucklos als auch unter Druck durchgeführt werden. Die Aufarbeitung des Reaktionsgemisches erfolgt mit an sich bekannten Methoden, wie Destillation.

## Beispiel 1

In einem Rührreaktor werden 316 Teile 4-Formyl-4-methyl-valeriansäuremethylester auf 100°C erhitzt, dann werden unter Rühren 150 Teile Sauerstoff (120 l) innerhalb von 4 Stunden durch eine Sinterglasfritte eingeleitet. Nach Beendigung der Reaktion wird das Reaktionsgemisch einer fraktionierenden Destillation unterworfen. Man erhält 180 Teile (79% der Theorie), $\gamma,\gamma$-Dimethyl-$\gamma$-butyrolacton (Kp. 80—82° C/16 mbar).

## Beispiel 2

Analog Beispiel 1 werden 186 Teile 4-Formyl-4-methylheptansäuremethylester mit 80 Teilen Sauerstoff innerhalb von 5 Stunden umgesetzt. Man erhält 86 Teile (61% der Theorie) $\gamma$-Methyl-$\gamma$-propyl-$\gamma$-butyrolacton (Kp. 117—119° C/20 mbar).

## Beispiel 3

Analog Beispiel 1 werden 172 Teile 4-Formyl-3,4-dimethylvaleriansäuremethylester mit 80 Teilen Sauerstoff innerhalb von 4 Stunden umgesetzt. Man erhält 91 Teile (71% der Theorie) $\beta,\gamma,\gamma$-Trimethyl-$\gamma$-butyrolacton (Kp. 94—96° C/16 mbar).

## Beispiel 4

Analog Beispiel 1 wird ein Gemisch aus 244 Teilen 4-Formyl-4-($\beta$-methoxycarbonylethyl)-hexansäuremethylester und 2,4 Teilen $CoCl_2 \cdot 6\,H_2O$ mit 100 Teilen Sauerstoff innerhalb von 5 Stunden umgesetzt. Anschließend wird mit Wasser gewaschen, mit wasserfreiem Magnesiumsulfat getrocknet und fraktionierend destilliert. Man erhält 113 Teile (56% der Theorie) $\gamma$-Ethyl-$\gamma$-($\beta'$-methoxycarbonylethyl)-$\gamma$-butyrolacton (Kp. 125° C/0,5 mbar).

**Patentansprüche**

1. Verfahren zur Herstellung von Butyrolactonen der allgemeinen Formel

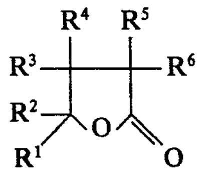

(I)

in der $R^1$ eine gegebenenfalls durch Cyan- Alkoxycarbonyl-, Alkylcarbonyloxy- oder Nitrogruppen oder Chloratome substituierte Alkyl- oder Phenylgruppe und die Reste $R^2$ bis $R^6$ Wasserstoffatome oder gegebenenfalls durch Cyan-, Alkoxycarbonyl-, Alkylcarbonyloxy, oder Nitrogruppen oder Chloratome substituierte Alkyl- oder Phenylgruppen bedeuten, wobei die Alkylgruppen $R^1$ bis $R^6$ 1—8 C-Atome aufweisen, dadurch gekennzeichnet, daß man $\gamma$-Formylcarbonsäureester der allgemeinen Formel

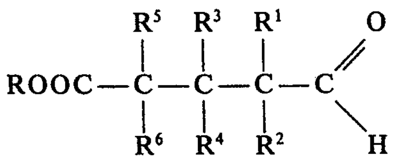

(II)

in der R eine niedere Alkylgruppe mit bis zu 4 C-Atomen bedeutet, bei 60 bis 150°C mit Sauerstoff behandelt.

2. Verfahren nach Anspruch 1, dadurch gekennzeichnet, daß man $\gamma$-Formylcarbonsäureester der Formel II verwendet, in der R eine Methyl-, Ethyl-, Propyl oder Butylgruppe, $R^1$ und $R^2$ Alkylgruppen mit 1 bis 6 C-Atomen, die durch Cyano-, Acetoxy-, Methoxycarbonyl-, Phenyl- oder Nitrogruppen oder durch Chloratome substituiert sein können und die Reste $R^3$ bis $R^6$ Wasserstoffatome bedeuten.

**Claims**

1. A process for the preparation of butyrolactones of the general formula

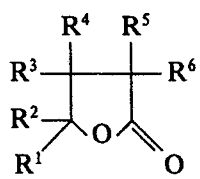

(I)

where $R^1$ is alkyl or phenyl optionally substituted by cyano, alkoxycarbonyl, alkylcarbonyloxy, nitro or chlorine, and $R^2$ to $R^6$ are hydrogen, or alkyl or phenyl optionally substituted by cyano, alkoxycarbonyl, alkylcarbonyloxy, nitro or chlorine, the alkyls $R^1$ to $R^6$ each having 1 to 8 carbon atoms, wherein a $\gamma$-formylcarboxylic acid ester of the general formula

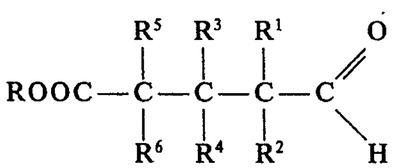

(II)

where R is lower alkyl of up to 4 carbon atoms, is treated with oxygen at 60 to 150° C.

2. A process as claimed in claim 1, wherein, in the $\gamma$-formylcarboxylic acid ester of the formula II, R is methyl, ethyl, propyl or butyl, $R^1$ and $R^2$ are alkyl of 1 to 6 carbon atoms, which may be substituted by cyano, acetoxy, methoxycarbonyl, phenyl, nitro or chlorine, and $R^3$ to $R^6$ are hydrogen.

**Revendications**

1. Procédé pour la préparation de butyrolactones de formule générale

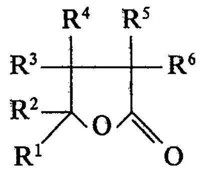

(I)

dans laquelle $R^1$ représente un groupe alcoyle ou phényle éventuellement substitué par des groupes cyano, alcoxycarbonyle, alcoylcarbonyloxy ou nitro, ou par des atomes de chlore, et les radicaux $R^2$ à $R^6$ sont des atomes d'hydrogène ou des groupes alcoyle ou phényle éventuellement substitués par des groupes cyano, alcoxycarbonyle, alcoylcarbonyloxy ou nitro ou par des atomes de chlore, les groupes alcoyle $R^1$ à $R^6$ comportant 1 à 8 atomes de C, caractérisé en ce qu'on traite par l'oxygène, à une température de 60 à 150° C, des esters d'acides $\gamma$-formylcarboxyliques de formule générale

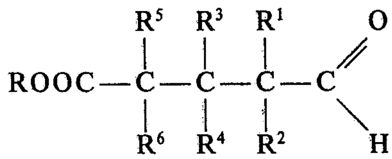

(II)

dans laquelle R représente un groupe alcoyle inférieur pouvant comporter jusqu'à 4 atomes de carbone.

2. Procédé selon la revendication 1, caractérisé en ce qu'on utilise des esters d'acides $\gamma$-formylcarboxyliques de formule II, dans lesquels R représente un groupe méthyle, éthyle, propyle ou butyle, $R^1$ et $R^2$ représentent des groupes alcoyle à 1—6 atomes de C, qui peuvent être substitués par des groupes cyano, acétoxy, méthoxy, carbonyle, phényle ou nitro ou par des atomes de chlore et les radicatx $R^3$ à $R^6$ sont des atomes d'hydrogène.